# EUROPÄISCHE PATENTSCHRIFT

(11) **EP 1 362 512 B1**
(45) Veröffentlichungstag und Bekanntmachung des Hinweises auf die Patenterteilung: **17.03.2010**
(21) Anmeldenummer: 03010067.1
(22) Anmeldetag: 03.05.2003
(51) Int. Cl.: A01N 37/44, A01N 33/04, A01N 33/12, A61L 2/18

(54) **Wässrige Desinfektionsmittel auf Basis von Alkylaminverbindungen und Aminopolycarbonsäuren**
Aqueous disinfectant based on alkylamines and aminopolycarboxylic acids
Désinfectants aqueux à base d'alkylamines et d' acides aminopolycarboxyliques

(30) Priorität: 17.05.2002 DE 10221982
(43) Veröffentlichungstag der Anmeldung: 19.11.2003
(73) Patentinhaber: Evonik Goldschmidt GmbH, 45127 Essen (DE)
(72) Erfinder: Hilmes, Wilfried, 45326 Essen (DE); Müller, Felix, Dr., 42555 Velbert (DE); Schramm, Herbert, 45289 Essen (DE)

(56) Entgegenhaltungen:
- EP-A- 0 612 470
- EP-A- 1 031 280
- WO-A-92/02489
- WO-A-98/30661

## Beschreibung

Die Erfindung betrifft wässrige Desinfektionsmittelkonzentrate auf Basis von Alkylaminverbindungen und Aminopolycarbonsäuren, daraus herstellbare Desinfektionsmittel sowie deren Verwendung.

Desinfektionsmittel werden zur Bekämpfung von Mikroorganismen in vielen Bereichen eingesetzt, wie beispielsweise Hände-, Operationsfeld- oder Wunddesinfektion, Instrumentendesinfektion, Desinfektion von Oberflächen, Textilien.

Bekannt sind beispielsweise Desinfektionsmittel, die als Wirkstoffe ein oder mehrere Alkyl-(poly)-amine in Kombination mit einer oder mehreren Komponenten beispielsweise aus der Gruppe der Alkohole, Glykolether, Tenside, Komplexbildner, Korrosionsinhibitoren und sonst üblichen Hilfs- und Zusatzstoffen enthalten.

So wird in der EP-A-0 612 470 ein Desinfektionsmittelkonzentrat bzw. ein Desinfektionsmittel vorgeschlagen, das Amin und Alkohol umfasst und dadurch gekennzeichnet ist, dass die Alkoholkomponente mindestens einen begrenzt wassermischbaren aliphatischen Glykolether mit 1 bis 4 Alkylenoxideinheiten umfasst und die Aminkomponente mindestens ein sekundäres und/oder tertiäres hydroxygruppenfreies Alkylamin, vorzugsweise N,N-Bis-(3-aminopropyl)-laurylamin, umfasst.

Aus der EP-A-0 333 143 ist ein flüssiger Desinfektionsreiniger bekannt, der als biozide Wirkstoffe tertiäre Alkylamine, insbesondere N,N-Bis-(3-aminopropyl)-laurylamin, und als Reinigungskomponente anionische Tenside enthält. Darüber hinaus kann dieses Reinigungsmittel als Lösungsmittel zusätzlich Wasser oder Alkohole mit bis zu 4 Kohlenstoffatomen wie Methanol, Ethanol usw. enthalten.

Gemäß der EP-A-0 343 605 wird der oben genannte Wirkstoff als tuberkulozides Desinfektionsmittel eingesetzt, welches neben einem Lösungsmittelgemisch von Wasser und einem Alkohol mit bis zu 4 Kohlenstoffatomen wie Methanol, Ethanol als Reinigungsmittel auch Tenside, quaternäre Ammoniumverbindungen sowie Komplexbildner enthalten kann.

Aus der WO-A-94/09105 ist ein reinigendes wässriges Desinfektionsmittel auf Basis von Umsetzungsprodukten aus
a) N-substituierten Propylendiaminen der Formel I:

   R₁-NH-CH₂-CH₂-CH₂-NH₂, (I),

   in der
   - R₁: für einen linearen Alkylrest mit 12 bis 14 Kohlen- stoffatomen steht, und
b) Verbindungen der Formel II

   R₂-O-CO-CH₂-CH₂-CH(NH₂) -COOH, (II),

   mit
   - R₂: Wasserstoffatom oder ein Alkylrest mit 1 bis 4 Koh- lenstoffatomen, bekannt, das dadurch gekennzeichnet ist, dass es als Lösungsvermittler Benzyl- und/oder 2-Phenoxyethanol und/oder 1-Phenoxypropanol enthält.

Gegenstand der EP-A-1 031 280 sind mikrobizide Mittel, enthaltend eine Wirkstoffkombination aus
a) primären oder sekundären Alkylaminen der allgemeinen Formel (I)

   R- [NR-(CH₂)ₓ-]_{y}-NR₂ (I),

   worin bedeuten
   - R =: C₈-C₁₈ unverzweigtes und gesättigtes Alkyl oder H,
   - x =: 1 bis 4,
   - y =: 1 bis 4 und
b) Amphotensiden auf der Basis von Alkylaminoessigsäuren der allgemeinen Formel (II)

   R¹NH- (Cₙ H₂ₙ-NH)ₘ(CH₂-COOH)ₒ (II),

   worin bedeuten
   - R¹=: C₈-C₁₈ unverzweigtes und gesättigtes Alkyl,
   - n =: 1 bis 3,
   - m =: 1 bis 3,
   - o =: 1 bis 2 und
c) aliphatischen Glykolethern der allgemeinen Formel (III)

   R-[O-(CH₂)ₓ]_{y}-O-H (III),

   mit
   - R =: C₁-C₄ Alkyl,
   - x =: 2 bis 4,
   - y =: 2 bis 4 und
d) Fettalkoholethoxylaten (Alkylpolyethylenglykolether) der allgemeinen Formel (IV)

   R (O-CH₂-CH₂)ₙOH (IV),

   mit
   - R =: C₆-C₁₈ Alkyl,
   - n =: 3 bis 20 und gegebenenfalls
   Komplexbildner, wie Phosphonate, Nitrilotriessigsäure (NTA), Ethylendiaminotetraessigsäure (EDTA), Polyasparaginsäure (PAPS), Methylglycindiisoessigsäure (MGDA), Citrate, Gluconate, Polycarboxylate und/oder Aminotris-(methylenphosphonsäure (ATMP).

WO 98/30 661 A1 offenbart Aminopolycarbonsäuren als Komplexierungsmittel in Oberflächenreinigern.

Nachteilig an den oben genannten bekannten Desinfektionsmitteln und -konzentraten ist, dass zur Herstellung klarer Lösungen, insbesondere bei Temperaturen um 10 °C, ein oder mehrere Lösungsvermittler in zum Teil beträchtlichen Mengen mitverwendet werden müssen und dass zur raschen und sicheren Keimabtötung relativ hohe Wirkstoffkonzentrationen benötigt werden.

Dabei besteht die Gefahr, dass aufgrund der Komponentenvielfalt durch eine ungünstige Kombination (beispielsweise: kationenaktive Verbindungen + anionische Tenside) zusätzlich eine partielle Unverträglichkeit mit gleichzeitig desaktivierender Wirkung auftreten kann und/oder die Bestandteile auf die zu desinfizierenden Oberflächen aufziehen und einen optisch und hygienisch unerwünschten Belag hinterlassen.

Aufgabe der vorliegenden Erfindung war es daher, diese Nachteile des Standes der Technik zu vermeiden und biozid wirkende Formulierungen zu finden, welche eine außer Wasser und der Wirkstoffkombination keine weiteren Bestandteile enthalten müssen und eine sichere Keimabtötung auch bei niedrigen Temperaturen ≤ 10 °C, bei möglichst kurzen Einwirkzeiten und in geringen Konzentrationen gegen eine Vielzahl von Keimen ermöglichen.

Diese Aufgabe wird gelöst durch wässrige Desinfektionsmittel auf Basis von Alkylaminverbindungen und Aminopolycarbonsäuren als synergistische Verstärkungsmittel der bioziden Wirkung.

Gegenstand der Erfindung sind daher wässrige Desinfektionsmittel auf Basis von Alkylaminverbindungen und Aminopolycarbonsäuren, welche dadurch gekennzeichnet sind, das sie als Aminopolycarbonsäuren mindestens eine der Verbindungen der allgemeinen Formel (I) 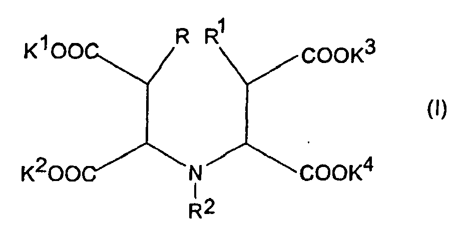 enthalten, worin bedeuten
- R, R¹: unabhängig voneinander H oder OH,
- R²: Wasserstoff oder ein Alkylrest mit 1 bis 18, vorzugs- weise 1 bis 8 C-Atomen,
- K¹, K², K³, K⁴: unabhängig voneinander ein gleiches oder ver- schiedenes Kation bedeuten, vorzugsweise aus der Gruppe umfassend Wasserstoff, Alkalimetallion, Ammo- niumion der allgemeinen Formel NR^{a}R^{b}R^{c}R^{d}, in der R^{a}R^{b}R^{c}R^{d} unabhängig voneinander Wasserstoff, gegebe- nenfalls hydroxysubstituierte Alkylreste mit 1 bis 18 C-Atomen bedeuten.

In keiner Weise vorhersehbar war die Tatsache, dass die Kombination der zuvor beschriebenen Komponenten eine synergistische Verschiebung des Wirksamkeitsspektrums hinsichtlich des Verhaltens solcher Mittel gegenüber Bakterien im 10°C-Anwendungsbereich ergab. Desinfektionsmittel auf Basis der erfindungsgemäß definierten Mittel lassen sich somit als hochwirksame Präparate zur Flächen- und Instrumentendesinfektion einsetzen.

Bei Zusatz von Verbindungen mit R, R¹, R² = H, R, R² = H, R¹ = OH, ergeben sich besonders stabile klare Desinfektionsmittel und Desinfektionsmittelkonzentrate, wobei die Desinfektionsmittellösungen auch bei Temperaturen um 10°C hohe biozide Wirksamkeit zeigen.

Bevorzugte Ausführungsformen der erfindungsgemäßen Desinfektionsmittel sind Gegenstand der Unteransprüche.

Die erfindungsgemäß mitverwendeten Alkylamine sind Verbindungen der allgemeinen Formel (II) und/oder (IIa)

R³-[NR⁴-(CH₂)ₓ-]_{y}-NR⁵R⁶ (II),

[R³-[NR⁴-(CH²)ₓ-]_{y}-NR⁵R⁶R¹⁰]⁺A- (IIa),

worin bedeuten
- R³: ein gegebenenfalls Mehrfachbindungen enthaltender, gege- benenfalls verzweigter Alkylrest mit 8 bis 22, vorzugsweise 12 bis 18 C-Atomen,
- R⁴,R⁵,R⁶,R¹⁰: unabhängig voneinander Wasserstoff, R³ oder gege- benenfalls amino- und/oder hydroxysubstituierte Alkylreste mit 2 bis 10 C-Atomen,
- A⁻ =: ein Anion,
- x =: 1 bis 4,
- y =: 0 bis 4
und/oder

Amphotenside auf der Basis von Alkylaminoessigsäuren der allgemeinen Formel (III)

R⁷-NR⁸- (CₙH₂ₙ-NR⁹)ₘ- (CH₂-COOK)ₐ (III),

worin bedeuten
- R⁷: ein gegebenenfalls Mehrfachbindungen enthaltender, gege- benenfalls verzweigter Alkylrest mit 8 bis 22, vorzugsweise 12 bis 18 C-Atomen,
- R⁸, R⁹: Wasserstoff oder bis zu (m + 1) der Rest - CH₂-COOK,
- K: Wasserstoff oder einer der Reste K¹, K², K³, K⁴,
- n =: 1 bis 3,
- m =: 1 bis 4,
- a =: 1 bis (m + 2).

Als Alkylamine der allgemeinen Formel (II) im Sinne der vorliegenden Erfindung werden vorzugsweise Oligotypen eingesetzt, d. h., 2 bis 4 aufeinanderfolgende N-Atome sind verbunden durch 1 bis 3 Alkylengruppen. 2 bis 3 aufeinanderfolgende N-Atome werden jedoch bevorzugt. Die C-Kettenverteilung der Alkylreste solcher Oligoamine liegt zwischen C₈-C₂₂. Die Alkylgruppen sind insbesondere unverzweigt und gesättigt. Die Alkylreste C₁₂/C₁₄ sind vorzugsweise dominant und zu 70/30 vertreten. In den DE-B-19 28 192, 19 44 255 und 21 15 549 wird der mikrobizide Charakter solcher Verbindungen allein bereits beschrieben.

Erfindungsgemäß bevorzugte Verbindungen der allgemeinen Formel (II) sind weiterhin solche, in denen R³ = Dodecylrest, y = 0 und R⁵, R⁶ -(CH₂)₃-NH₂ sind. Diese Verbindungen sind handelsübliche Produkte und werden als N,N-Bis-(3-aminopropyl)-laurylamin unter der Bezeichnung "Lonzabac^{®} 12" von der Firma Lonza Ltd., Basel, Schweiz oder mit R³ = Talgfettalkylrest als "Hoe S 3119" von der Firma Clariant AG, Frankfurt angeboten.

Weitere Verbindungen sind die von der Firma Clariant angebotenen "Genamin^{®}-Typen wie Genamin^{®} CCP100D (R³ = C_{12/14}-Alkyl, R⁴, R⁵, R⁶ = H, x = 3, y = 1) oder Genamin^{®} LAP100D. (R³ = C_{10/16}-Alkyl, R⁴, R⁵, R⁶ = H, x = 3, y = 1).

Amphotenside der allgemeinen Formel (III) sind vorzugsweise Reaktionsprodukte aus Alkyloligoaminen mit Halogenessigsäure. Sie haben 2 bis 5 aufeinanderfolgende N-Atome, verbunden durch 1 bis 3 Alkylengruppen, vorzugsweise jedoch 2 bis 3 aufeinanderfolgende N-Atome. Die C-Kettenverteilung der Alkylreste solcher Amphotenside liegt insbesondere zwischen C₈-C₂₂. Sie sind vorzugsweise unverzweigt und gesättigt. Die Alkylreste C_{12/14} sind vorzugsweise dominant und zu 70/30 vertreten. Ihre Herstellung ist durch die DE-B-19 51 156 und 19 28 192 beschrieben.

Erfindungsgemäß bevorzugte Verbindungen der allgemeinen Formel (II) sind Amphotenside auf Basis Alkylpropylendiamin Tego^{®} 2000 der Firma Goldschmidt, Essen.

Die Alkylamin-Verbindungen der allgemeinen Formel (II) und die Amphotenside der allgemeinen Formel (III) können allein oder als Mischung miteinander oder untereinander mitverwendet werden. Ihr Mischungsverhältnis untereinander ist unkritisch in breiten Bereichen varierbar.

Der Anteil an Alkylamin (II und/oder III) im wässrigen Konzentrat kann insgesamt bis 35 Gew.-% betragen, bezogen auf die Gesamtformulierung und wenn keine sonstigen Zusätze wie Alkohole, Glykolether, etc. mitverwendet werden, bis 50 Gew.-% bei Formulierungen mit Zusätzen. Die Gesamtmenge der Verbindungen der allgemeinen Formeln (II) und/oder (III) im Konzentrat wird durch ihre von der Praxis vorgegebenen anwendungstechnischen Eigenschaften wie beispielsweise ihre Viskosität bei Raumtemperatur begrenzt und liegt bei dem erfindungsgemäß bevorzugten pH-Wert von ca. 8 bis 9 bei etwa 35 Gew.-%.

Höhere Konzentrationen sind möglich, sofern erforderlich oder erwünscht und können von dem Fachmann anhand seiner allgemeinen Erfahrung und Kenntnis durch einfache orientierende Versuche erzielt werden.

Die Verbindungen der allgemeinen Formel (I) können mit den Verbindungen der allgemeinen Formeln [(II) + (III)] im Gew.-Verhältnis 1 : 3 bis 1 : 14, vorzugsweise 1 : 5 bis 1 : 11, eingesetzt werden.

Die gebrauchsfertige Lösung, die gegebenenfalls durch Verdünnen des Konzentrats herzustellen ist, enthält die synergistische Wirkstoffkombination in einer Konzentration von 0,05 bis 8 Gew.-%, vorzugsweise in einer Konzentration von 0,1 bis 4 Gew.-%.

Als Lösungsmittel findet vorzugsweise Wasser Verwendung. Gegebenenfalls kann jedoch auch ein wasserhaltiges Lösungsmittelgemisch eingesetzt werden.

Wird ein Lösungsmittelgemisch eingesetzt, so enthält dieses vorzugsweise einen oder mehrere wasserlösliche organische Lösungsmittel, vorzugsweise aus den Gruppen der Alkohole mit 1 bis 4 C-Atomen und/oder Diglykolether der allgemeinen Formel (IV)

R^{I}- (OR^{II})ₙ-OH

worin
- R^{I}: OH oder ein gegebenenfalls verzweigter Alkylrest mit 1 bis 4 C-Atomen,
- R^{II}: ein gegebenenfalls verzweigter Alkylrest mit 2 bis 4 C- Atomen und
- n: eine ganze Zahl zwischen 1 und 4 bedeutet, wie beispiels- weise Methanol, Ethanol, Propanol, Isopropanol, tert.-Buta- nol, Ethylenglykol, Propylenglykol, Butylenglykol, Diethy- lenglykol, Dipropylenglykol, Diethylenglykolmonomethyl- ether, Diethylenglykolmonoethylether, Diethylenglykolmono- propylether und Diethylenglykolmonobutylether.

Weiterhin können gewünschtenfalls alle auf diesem Gebiet üblichen Hilfs- und Zusatzstoffe beispielsweise Tenside wie Alkylpolyglykoside mit vorzugsweise 8 bis 22 C-Atomen im Alkylrest; Umsetzungsprodukte aus Fettalkoholen, Fettsäuren, Fettaminen, Fettsäureamiden mit Ethylenoxid oder Propylenoxid, insbesondere die Anlagerungsprodukte von 3 bis 16 Mol Ethylenoxid an Kokos- oder Talgfettalkohole, an Oleylalkohol sowie an Mono-, Di- oder Trialkylphenole sowie an Monoalkylcyclohexanole mit 6 bis 14 Kohlenstoffatomen in den Alkylresten; Esterquats auf Basis von Umsetzungsprodukten aus Triethanolamin und Fettsäuren; Trialkylammoniumverbindungen; Amphotenside; Komplexbildner wie beispielsweise die Alkali-, vorzugsweise die Natriumsalze der Methandiphosphonsäure, Hydroxyethan-1,1-diphosphonsäure, 1-Aminoethan-1,1-diphosphonsäure, Aminotrimethylenphosphonsäure), Ethylendiamintetra-(methylenphosphonsäure), Diethylentriamin-penta(methylenphosphonsäure), 2-Phosphonobutan-1,2,4-tricarbonsäure, Nitrilotriessigsäure (NTA), Ethylendiamintetraessigsäure und Hydroxyethyl-ethylendiamintriessigsäure; Korrosionsschutzmittel aus den Gruppen der Phosphonsäuren, Aminocarbonsäuren und deren Salzen, insbesondere deren Alkalisalzen, Farbstoffe, Puffer, Entschäumer, Stabilisatoren und/oder Duftstoffe in den bekannten Anteilen mitverwendet werden.

Diese Zusätze können in Mengen von etwa 0 bis 35, vorzugsweise bis etwa 20 Gew.-%, bezogen auf die Gesamtformulierung, eingesetzt werden.

Von Bedeutung ist die Einstellung des pH-Wertes der erfindungsgemäßen Mittel. Er sollte vorzugsweise im alkalischen Bereich liegen und sich etwa von pH 7 bis 11, vorzugsweise 8 bis 9, erstrecken. Höhere pH-Werte sind bei der gegebenen Wirkstoffkombination nicht möglich und führen zu chemischen Inkompatibilitäten. Tiefere pH-Werte führen zu deutlichen Wirksamkeitsverlusten im angegebenen Wirkungsbereich der erfindungsgemäßen Mittel. Zum Einstellen des pH-Wertes sind die auf diesem Gebiet gebräuchlichen anorganischen und vorzugsweise organischen Säuren verwendbar, insbesondere Essigsäure. Geeignet sind jedoch auch Ameisensäure oder auch mehrfunktionelle Säuren, wie beispielsweise Citronensäure, Milchsäure oder Weinsäure.

Gegenüber den aus dem Stand der Technik bekannten Desinfektionsmittelkonzentraten bzw. Desinfektionsmitteln, die lediglich die sekundären und/oder tertiären Amine der allgemeinen Formel (II) und/oder Amphoacetate der allgemeinen Formel (III) sowie deren Formulierungen mit Alkoholen und den (begrenzt wassermischbaren) Glykolethern als Lösungsvermittler enthalten, zeichnet sich das erfindungsgemäße Desinfektionsmittelkonzentrat durch bessere anwendungstechnische Eigenschaften wie z.B. klare Löslichkeit auch bei niedrigen Temperaturen, eine niedrigere Viskosität und höhere Lagerstabilität aus.

Weiterhin zeichnen sich das erfindungsgemäße Desinfektionsmittelkonzentrat bzw. das Desinfektionsmittel durch Emissionsarmut, d.h. Geruchsarmut, aus und sie weisen keinen Flammpunkt auf.

Die biozide Wirksamkeit beispielsweise gegenüber Pseudomonas aeruginosa, Staphylococcus aureus, Enterococcus hirae oder Escherichia coli wird im folgenden anhand von Beispielen näher erläutert.

### Beispiele:

Für die Ermittlung der Wirksamkeit gegenüber Gram⁻/Gram⁺-Bakterien im Anwendungsbereich 10 °C erfolgte die Prüfung gemäß den Richtlinien der Europäischen Union -"Quantitativer Suspensionsversuch zur Bestimmung der bakteriziden Wirkung chemischer Desinfektionsmittel und Antiseptika in den Bereichen Lebensmittel, Industrie, Haushalt und öffentlichen Einrichtungen"- (CEN EN 1276, Juni 1997). Es handelt sich hier um quantitative Suspensionsversuche mit und ohne organische Belastung.

Der CEN EN 1276 fordert eine Konzentration-Zeit-Relation von 5 log-Einheiten innerhalb von 5 Minuten. Geprüft wird mit und ohne Belastung bei 20 °C oder wahlweise alternativer Temperatur beispielsweise der kritischeren Temperatur von 10 °C. Alle Versuche erfolgen unter Verwendung entsprechender Enthemmersysteme.

Es wird die Fähigkeit eines Produktes bestimmt, die Anzahl lebender vegetativer Bakterienzellen bestimmter Referenzstämme unter Bedingungen dieser Europäischen Norm zu vermindern. Diese Anforderungen werden nachfolgend grob skizziert:

| | | |
|---|---|---|
| Als Testorganismen: | Pseudomonas aeruginosa | ATCC 15442 |
| | Escherichia coli | ATCC 10536 |
| | Staphylococcus aureus | ATCC 6358 |
| | Enterococcus hirae | ATCC 10541 |
| Weitere Bedingungen: | Temperatur | 10 °C ± 1 °C |
| | Kontaktzeit | 5 min + 10 s |
| | hohe Belastung | 0,3 % Rinderalbumin |
| | Lösungen/Testlsg. | Wasser mit 300 ppm CaCO₃ |

Die Ergebnisse sind nachfolgend dokumentiert.

### Beispiel 1 (Vergleich):

Durch vermischen der entsprechenden Bestandteile wurde in einem geeigneten Gefäß eine Formulierung (Konzentrat) erstellt, bestehend aus
22 Gew.-% der allgemeinen Formel (II) mit R⁷= C_{12/14}-Alkyl,
m = 1,
a = 1,
mit 60 %iger Essigsäure auf pH 8,4 eingestellt, und
ad 100 Gew.-% Wasser.

### Beispiel 2 (Erfindung):

Wie in Beispiel 1 wurde eine Formulierung (Konzentrat) erstellt, bestehend aus
20 Gew.-% der allgemeinen Formel (II) mit R⁷ = C_{12/14} -Alkyl,
m = 1,
a = 1, und
2 Gew.-% Imidodibernsteinsäure (100 %ig) und
mit 60 %iger Essigsäure auf pH 8,4 eingestellt, und
ad 100 Gew.-% Wasser.

### Beispiel 3 (Vergleich):

Wie in Beispiel 1 wurde eine Formulierung (Konzentrat) erstellt, bestehend aus
22 Gew.-% Imidodibernsteinsäure (100 %ig) und
mit 60 %iger Essigsäure auf pH 8,4 eingestellt, und
ad 100 Gew.-% Wasser.

### Beispiel 4 (Vergleich DE-A-199 08 553, Beispiel 1) :

Wie in Beispiel 1 wurde eine Formulierung (Konzentrat) erstellt, bestehend aus
6 Gew.-% der allgemeinen Formel (II) mit R⁷ = C_{12/14}-Alkyl,
m = 1,
a = 1,
4 Gew.-% Alkylpropylendiamin (Genamin^{®} LAP100D),
6 Gew.-% Dipropylenglykolmonomethylether (Dowano^{®} DPM),
4 Gew.-% PEG-12-Isotridecanol (TEGO^{®} Alkanol TD12),
9 Gew.-% Nitrilotriessigsäure
ad 100 Gew.-% Wasser mit Zusatzstoffen (aliphatischer Alkohol < 2 %, Essigsäure zur pH-Einstellung auf pH 9,8).

### Beispiel 5 (Erfindung):

Wie in Beispiel 1 wurde eine Formulierung (Konzentrat) erstellt, bestehend aus
20 Gew.-% der allgemeinen Formel (II) mit R³ = C_{12/14} -Alkyl,
y = 1,
R⁴,R⁵,R⁶ = H, und
2 Gew.-% Imidodibernsteinsäure (100 %ig) und
mit 60 %iger Essigsäure auf pH 8,4 eingestellt, und
ad 100 Gew.-% Wasser.

### Durchführung:

3 verschiedene Verdünnungen aus den Beispielen 1 bis 4 wurden nach EN 1276 getestet. Eine Reduzierung von ≥ 5 log-Stufen wird gefordert. Die folgende Tabelle zeigt die entsprechenden Konzentrationen, die bei 10 °C und höherer Belastung, die Anforderung des EN 1276 erfüllen.

| | Die sichere Konzentration (% Beispiel X), bei welcher eine Reduktion von ≥ 5 log-Stufen erreicht wurde, ist angegeben. | | | | |
|---|---|---|---|---|---|
| | Beispiel 2E* | Bei-spiel 1V* | Beispiel 3V | Beispiel 4V | Beispiel 5E |
| P. aeruginosa | 0,5 % | 2,0 % | > 4 % | 1,0 % | 0,5 |
| ATCC 15442 | | | | | |
| E. coli | 0,5 % | 0,5 % | > 4 % | 0,5 % | 0,5 |
| ATCC 10536 | | | | | |
| S. aureus | 0,5 % | 0,5 % | > 4 % | 0,5 % | 0,5 |
| ATCC 6358 | | | | | |
| E. hirae | 0,5 % | 0,5 % | > 4 % | 0,5 % | 0,5 |
| ATCC 10541 | | | | | |
| Testkonzentration | 0,5 % | 2,0 % | > 4 % | 1,0 % | 0,5 |

| | | | | | |
|---|---|---|---|---|---|
| * E = erfindungsgemäßes Beispiel; * V = Vergleichsbeispiel | | | | | |

Anhand der Tabelle zeigt sich, dass durch die erfindungsgemäße Kombination der Komponeten in Beispiel 2 und 5, eine sprunghafte Wirksamkeitssteigerung gegenüber dem in der Praxis besonders kritischen Keimen Pseudomonas aeruginosa erzielt wurde.

Diese Wirkung wird überraschenderweise in einem praxisgerechten niedrigen pH-Bereich von 8 bis 9 und ohne den Einsatz von weiteren Hilfsstoffen erreicht.

## Patentansprüche

1. Wässrige Desinfektionsmittel auf Basis von Alkylaminverbindungen und Aminopolycarbonsäuren, die **dadurch gekennzeichnet sind, dass** sie als Aminopolycarbonsäuren mindestens eine der Verbindungen der allgemeinen Formel (I) 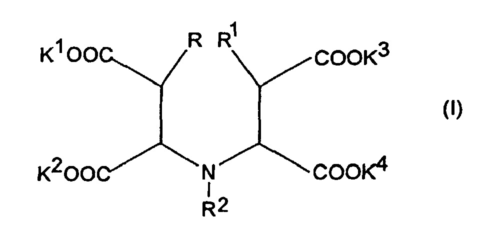 enthalten, worin bedeuten
R, R¹ unabhängig voneinander H oder OH,
R² Wasserstoff oder ein Alkylrest mit 1 bis 18, vorzugsweise 1 bis 8 C-Atomen,
K¹, K², K³, K⁴ unabhängig voneinander ein gleiches oder verschiedenes Kation bedeuten, vorzugsweise aus der Gruppe umfassend Wasserstoff, Alkalimetallion, Ammoniumion der allgemeinen Formel NR^{a}R^{b}R^{c}R^{d}, in der R^{a}R^{b}R^{c}R^{d} unabhängig voneinander Wasserstoff, gegebe- nenfalls hydroxysubstituierte Alkylreste mit 1 bis 18 C-Atomen bedeuten.

2. Wässrige Desinfektionsmittel gemäß Anspruch 1, **dadurch gekennzeichnet, dass** in der Verbindung der allgemeinen Formel (I) R, R¹, R² = H und/oder R, R² = H, R¹ = OH sind.

3. Wässrige Desinfektionsmittel gemäß einem der Ansprüche 1 oder 2, **dadurch gekennzeichnet, dass** als Alkylaminverbindungen eine oder mehrere der Verbindungen der allgemeinen Formel (II) und/oder (IIa)
R³-[NR⁴-(CH₂)ₓ-]_{y}-NR⁵R⁶ (II)
[R³-[NR⁴-(CH₂)ₓ-]_{y}-NR⁵R⁶R¹⁰)⁺A⁻ (IIa),
mitverwendet werden, worin bedeuten
R³ ein gegebenenfalls Mehrfachbindungen enthaltender, gege- benenfalls verzweigter Alkylrest mit 8 bis 22, vorzugsweise 12 bis 18 C-Atomen,
R⁴,R⁵,R⁶,R¹⁰ unabhängig voneinander Wasserstoff, R³ oder gege- benenfalls amino- und/oder hydroxysubstituierte Alkylreste mit 2 bis 10 C-Atomen,
A⁻ = ein Anion,
x = 1 bis 4,
y = 0 bis 4.

4. Wässrige Desinfektionsmittel gemäß einem der Ansprüche 1 bis 3, **dadurch gekennzeichnet, dass** als Aminverbindungen eine oder mehrere der Amphotenside der allgemeinen Formel (III)
R⁷-NR⁸-(CₙH₂ₙ-NR⁹)ₘ-(CH₂-COOK)ₐ (III)
mitverwendet werden, worin bedeuten
R⁷ ein gegebenenfalls Mehrfachbindungen enthaltender, gegebenenfalls verzweigter Alkylrest mit 8 bis 22, vorzugsweise 12 bis 18 C-Atomen,
R⁸, R⁹ Wasserstoff oder bis zu (m+1) der Rest -CH₂-COOK,
K Wasserstoff oder einer der Reste K¹, K², K³, K⁴,
n = 1 bis 3,
m = 1 bis 4,
a = 1 bis (m + 2).

5. Wässrige Desinfektionsmittel gemäß einem der Ansprüche 1 bis 4, **dadurch gekennzeichnet, dass** die Verbindungen der allgemeinen Formel (I) mit den Verbindungen der allgemeinen Formeln [(II) + (III)] im Gew.-Verhältnis 1 : 3 bis 1 : 14, vorzugsweise 1 : 5 bis 1 : 11, eingesetzt werden.

6. Desinfektionsmittelkonzentrat nach einem der Ansprüche 1 bis 5, enthaltend 0,3 bis 17,0 Gew.-% mindestens eine der Verbindungen der allgemeinen Formel (I) und 5,0 bis 50,0 Gew.-% mindestens eine der Verbindungen der allgemeinen Formeln (II) und/oder (III) und gegebenenfalls 0,0 bis 30,0 Gew.-% übliche Hilfs- und Zusatzstoffe und ad 100 Gew.-% Wasser.

## Claims

1. Aqueous disinfectants based on alkylamine compounds and aminopolycarboxylic acids which are **characterized in that** they comprise, as aminopolycarboxylic acids, at least one of the compounds of the general formula (I) 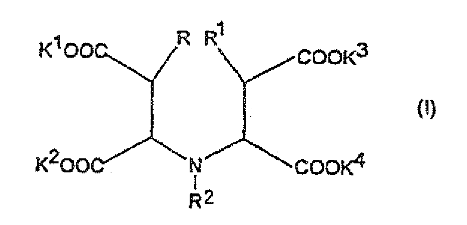 in which
R, R¹, independently of one another, are H or OH,
R² is hydrogen or an alkyl radical having 1 to 18, preferably 1 to 8, carbon atoms,
K¹, K², K³, K⁴, independently of one another, are an identical or different cation, preferably from the group comprising hydrogen, alkali metal ion, ammonium ion of the general formula NR^{a}R^{b}R^{c}R^{d}, in which R^{a}R^{b}R^{c}R^{d}, independently of one another, are hydrogen, optionally hydroxy-substituted alkyl radicals having 1 to 18 carbon atoms.

2. Aqueous disinfectants according to Claim 1, **characterized in that** in the compound of the general formula (I), R, R¹, R² = H and/or R, R² = H, R¹ = OH.

3. Aqueous disinfectants according to one of Claims 1 or 2, **characterized in that**, as alkylamine compounds, one or more of the compounds of the general formula (II) and/or (IIa)
**R³-[NR⁴-(CH₂)ₓ-]_{y}-NR⁵R⁶** **(II),**
**[R³-[NR⁴-(CH₂)ₓ-]_{y}-NR⁵R⁶R¹⁰]⁺A⁻** **(IIa),**
are co-used, in which
R³ is an optionally multiple-bond-containing, optionally branched alkyl radical having 8 to 22, preferably 12 to 18, carbon atoms,
R⁴, R⁵, R⁶, R¹⁰, independently of one another, are hydrogen, R³ or optionally amino- and/or hydroxy-substituted alkyl radicals having 2 to 10 carbon atoms,
A⁻ = an anion,
x = 1 to 4,
y = 0 to 4.

4. Aqueous disinfectants according to one of Claims 1 to 3, **characterized in that**, as amine compounds, one or more of the amphoteric surfactants of the general formula (III)
**R⁷-NR⁸-(CₙH₂ₙ-NR⁹)ₘ-(CH₂-COOK)ₐ** **(III)**
are co-used, in which
R⁷ is an optionally multiple-bond-containing, optionally branched alkyl radical having 8 to 22, preferably 12 to 18, carbon atoms,
R⁸, R⁹ is hydrogen or up to (m+1) the radical -CH₂-COOK,
K is hydrogen or one of the radicals K¹, K², K³, K⁴,
n = 1 to 3,
m = 1 to 4,
a = 1 to (m + 2).

5. Aqueous disinfectants according to one of Claims 1 to 4, **characterized in that** the compounds of the general formula (I) are used with the compounds of the general formulae [(II) + (III)] in the weight ratio 1 : 3 to 1 : 14, preferably 1 : 5 to 1 : 11.

6. Disinfectant concentrate according to one of Claims 1 to 5, comprising 0.3 to 17.0% by weight of at least one of the compounds of the general formula (I) and 5.0 to 50.0% by weight of at least one of the compounds of the general formulae (II) and/or (III) and optionally 0.0 to 30.0% by weight of customary auxiliaries and additives and ad 100% by weight water.

## Revendications

1. Désinfectants aqueux à base de composés alkylamine et d'acides aminopolycarboxyliques, qui sont **caractérisés en ce qu'**ils contiennent en tant qu'acides aminopolycarboxyliques au moins un des composés de formule générale (I) 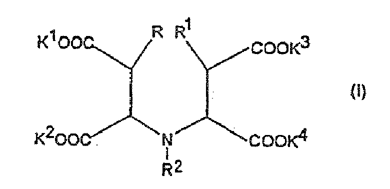 dans laquelle
R, R¹ représentent, indépendamment l'un de l'autre, H ou OH,
R² représente un atome d'hydrogène ou un radical alkyle ayant de 1 à 18, de préférence de 1 à 8 atomes de carbone,
K¹ , K², K³, K⁴ représentent, indépendamment les uns des autres, un cation, le même ou différent, choisi de préférence dans le groupe comprenant un atome d'hydrogène, un ion de métal alcalin, l'ion ammonium de formule générale NR^{a}R^{b}R^{c}R^{d}, dans laquelle
R^{a}R^{b}R^{c}R^{d} représentent, indépendamment les uns des autres, un atome d'hydrogène, des radicaux alkyle ayant de 1 à 18 atomes de carbone, éventuellement substitués par hydroxy.

2. Désinfectants aqueux selon la revendication 1, **caractérisés en ce que** dans le composé de formule générale (I) R, R¹, R² = H et/ou R, R² = H, R¹ = OH.

3. Désinfectants aqueux selon la revendication 1 ou 2, **caractérisés en ce qu'**on utilise en même temps en tant que composés alkylamine un ou plusieurs des composés de formule générale (II) et/ou de formule générale (IIa)
R³-[NR⁴-(CH₂)ₓ-]_{y}-NR⁵R⁶ (II),
[R³-[NR⁴-(CH₂)ₓ-]_{y}-NR⁵R⁶R¹⁰]⁺A⁻ (IIa),
dans lesquelles
R³ représente un radical alkyle ayant de 8 à 22, de préférence de 12 à 18 atomes de carbone, éventuellement ramifié, contenant éventuellement des liaisons multiples,
R⁴, R⁵, R⁶, R¹⁰ représentent, indépendamment les uns des autres, un atome d'hydrogène, R³ ou des radicaux alkyle ayant de 2 à 10 atomes de carbone, éventuellement substitués par amino et/ou hydroxy,
A représente un anion,
x va de 1 à 4,
y va de 0 à 4.

4. Désinfectants aqueux selon l'une quelconque des revendications 1 à 3, **caractérisés en ce qu'**on utilise en même temps en tant que composés de type amine un ou plusieurs des tensioactifs amphotères de formule générale (III)
R⁷-NR⁸-(CₙH₂ₙ-NR⁹)ₘ-(CH₂-COOK)ₐ (III)
dans laquelle
R⁷ représente un radical alkyle ayant de 8 à 22, de préférence de 12 à 18 atomes de carbone, éventuellement ramifié, contenant éventuellement des liaisons multiples,
R⁸, R⁹ représentent des atomes d'hydrogène ou jusqu'à (m+1) fois le radical -CH₂-COOK,
K représente un atome d'hydrogène ou l'un des radicaux K¹, K², K³, K⁴,
n = 1 à 3,
m = 1 à 4,
a = 1 à (m + 2).

5. Désinfectants aqueux selon l'une quelconque des revendications 1 à 4, **caractérisés en ce que** les composés de formule générale (I) sont utilisés avec les composés de formules générales [(II) + (III)] en un rapport pondéral de 1:3 à 1:14, de préférence de 1:5 à 1:11.

6. Concentré de désinfectant selon l'une quelconque des revendication 1 à 5, contenant de 0,3 à 17,0 % en poids d'au moins l'un des composés de formule générale (I) et de 5,0 à 50,0 % en poids d'au moins l'un des composés de formule générale (II) et/ou de formule générale (III) et éventuellement de 0,0 à 30,0 % en poids d'additifs et adjuvants usuels et de l'eau en complément à 100 % en poids.
